# EUROPEAN PATENT SPECIFICATION

(11) **EP 3 556 355 B1**
(45) Date of publication and mention of the grant of the patent: **29.12.2021**
(21) Application number: 19169226.8
(22) Date of filing: 15.04.2019
(51) Int. Cl.: A61K 9/14, A61K 9/16, A61K 9/20, A61K 31/00, A61K 31/7048, A61P 25/00

(54) **PARTICLES CONTAINING AMORPHOUS EMPAGLIFLOZIN, PROCESS FOR THEIR PREPARATION AND PHARMACEUTICAL PREPARATION**
PARTIKEL MIT AMORPHEM EMPAGLIFLOZIN, VERFAHREN ZU DEREN HERSTELLUNG UND PHARMAZEUTISCHES PRÄPARAT
PARTICULES CONTENANT DE L'EMPAGLIFLOZINE AMORPHE, LEUR PROCÉDÉ DE PRÉPARATION ET PRÉPARATION PHARMACEUTIQUE

(30) Priority: 18.04.2018 CZ 20180188
(43) Date of publication of application: 23.10.2019
(73) Proprietor: Zentiva, K.S., 10200 Praha 10 - Dolni Mecholupy (CZ)
(72) Inventor: Kovalcik, Pavel, 10200 Praha 10 (CZ)
(74) Representative: Hartvichova, Katerina

(56) References cited:
- WO-A1-2016/051368
- WO-A1-2017/203457
- CN-A- 106 880 595

## Description

### Field of Art

The present invention relates to particles containing amorphous empagliflozin and a pharmaceutically acceptable polymer, and to a process for preparation thereof by spray drying method.

### Background Art

Empagliflozin, or (1S)-1,5-anhydro-1-(4-chloro-3-{4-[(3S)-tetrahydrofuran-3-yloxy]benzyl}phenyl)-D-glucitol, is a potent inhibitor of sodium-glucose co-transporter type 2 (SGLT-2), and is therefore used to treat type 2 diabetes. It is currently approved for the treatment of type 2 diabetes and improvement of blood sugar control.

The empagliflozin molecule, which belongs to glucopyranosyl-substituted benzene derivatives, was first described in International Patent Application WO 2005/092877 and has the following structural formula:

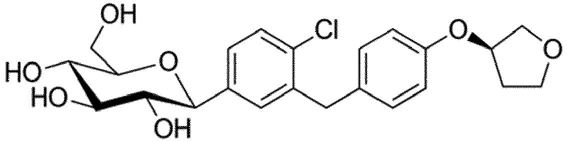

Depending on the preparation conditions, empagliflozin may form a crystalline form or an amorphous form. The amorphous form is highly unstable. These forms differ in their physical properties, in particular solubility and bioavailability. The first mention of the crystalline form of empagliflozin can be found in patent application WO 2006/117359. A pharmaceutical composition comprising the crystalline form of empagliflozin was then described in patent application WO 2010/092126. The original product, marketed under the trade name Jardiance, contains crystalline empagliflozin and is commercially available in a dosage form having a total strength of 10 or 25 mg of the active ingredient with the recommended dosage once daily. The dosage form is an immediate release film-coated tablet in which the particles have the size D(0.9) ≤ 200 µm as measured by dynamic light scattering (DLS) method. The particle size is a critical factor for the dissolution rate of empagliflozin, despite the fact that, according to the biopharmaceutical classification system, empagliflozin is included in the BCS III group, i.e. substances well soluble but poorly absorbable. For such substances, a person skilled in the art would expect that particle size does not have a major impact on their bioavailability. Surprisingly, however, the solubility and thus the bioavailability of crystalline empagliflozin is dependent on particle size as described in WO 2010/092126. Therefore, in the preparation of dosage forms containing crystalline empagliflozin, the particle size should be closely monitored, since any deviation may strongly affect the dissolution properties of the final product.

The solubility of empagliflozin can also be improved by using an amorphous form instead of the crystalline form. However, amorphous empagliflozin alone is highly unstable and recrystallizes virtually immediately. WO 2016/051368 describes the stabilization of amorphous empagliflozin by complexation with cyclodextrin. The process consists of dissolving crystalline empagliflozin in a solvent, adding cyclodextrin and isolating the solid dispersion of empagliflozin and cyclodextrin after solvent evaporation. Methanol was used as the solvent, at an elevated temperature of 60 °C. Such a process entails considerable safety risks associated with the fact that methanol is toxic and, moreover, a very volatile substance. Czech patent application PV 2015-279 describes the preparation of an amorphous form of empagliflozin with polymers by evaporation of the solvent by rotary vacuum evaporation or lyophilization. However, such preparation process results in a thin film of the product which has to be subjected to further processing steps, e.g. grinding, and yet the product form obtained does not have fully suitable physical properties for the preparation of solid pharmaceutical forms. Moreover, evaporation of the solvent is very time-consuming and leads to inhomogeneity of the resulting film.

Czech patent application PV 2017-152 describes the preparation of an amorphous form of empagliflozin with pharmaceutically acceptable polymers by hot-melt extrusion. However, hot-melt extrusion is carried out at high temperatures, thus causing degradation of some of the empaglifozin used and decreasing the yield of the preparation. Moreover, the empagliflozin thus prepared does not have fully suitable physical properties for the preparation of solid pharmaceutical forms (see, for example, the need to use flow enhancers in examples of pharmaceutical formulations in PV 2017-152), and eventually must be subjected to a milling step. WO 2017/203457 discloses a spray-drying process for the preparation of powdery solid solutions of empagliflozin and a polymer, such as polyethylene glycol, cellulose (derivatives) and polyvinylpyrrolidone.

Therefore, there is a persisting need in the art for an easy, economically advantageous process for preparing amorphous empagliflozin with good solubility and stability.

### Disclosure of the Invention

Within the framework of the present invention, the inventors have found that amorphous empagliflozin in combination with a pharmaceutically acceptable polymer can produce highly stable solid solutions by spray drying, wherein the resulting particles have excellent physical properties for the preparation of solid pharmaceutical forms. The invention is defined by the claims.

Compared to hot-melt extrusion, spray drying is performed at lower temperatures, so there is no risk of empagliflozin degradation. Moreover, the obtained material is in the form of spherical particles with excellent flow properties and can be directly mixed with other excipients. Spray drying results in spherical particles of relatively narrow particle distribution. The specific particle size can be easily influenced by the conditions during the spray drying process, including, in particular, drying gas temperature, drying gas flow rate, dried material flow rate and nozzle atomization pressure. If constant conditions during the process are ensured, particles of approximately the same size are always obtained.

Particle sphericity additionally provides advantageous flow properties and the particles can thus be easily formulated into the final product in the form of a tablet or a capsule. Thus, for example, the milling of an extrudate that would be obtained when using hot-melt extrusion technology or the grinding of a product that would be obtained by evaporating the solvent is eliminated. By milling, a wide particle size distribution would be obtained, for example 1-200 µm. Moreover, the sphericity of the resulting ground particles cannot be ensured by grinding. In the spray-dried product, empagliflozin is enclosed in the polymer matrix in the form of a solid solution, thereby reducing its undesirable adhesive properties. At the same time, the dustiness and potential exposure to empagliflozin during tableting, capsule filling or powder packaging are also reduced. This is very important, as it is a substance classified as OEB 3 (Occupational Exposure Band). This classification expresses the occupational exposure range and, for empagliflozin, the preliminary value is specifically set at 20 µg/m³ (OEL, Occupational Exposure Limit). Empagliflozin in the form of solid solution can be easily formulated with excipients into a pharmaceutical composition. The formulation thus prepared is highly stable, there is no subsequent recrystallization or degradation of empagliflozin, and thus no special drug storage conditions are required.

For example, particle size can be measured by dynamic light scattering and/or image analysis of particle images. In this document, image analysis of the scanning electron microscope images was used to determine particle size.

The present invention provides particles (i.e., a particular composition) containing amorphous empagliflozin and a pharmaceutically acceptable polymer having a size as determined by electron microscopy image analysis expressed as D(0.9) less than or equal to 100 µm, preferably less than or equal to 50 µm, most preferably less than or equal to 20 µm. The particles are obtainable by a spray-drying process as defined in the claims.

D(0.9) is the 90th quantile of particle size, the value of D(0.9) indicates the range or limit of the particle size in which are 90 vol. % of all particles in the system.

Also within the framework of the present invention, a stabilized amorphous empagliflozin with a narrow spherical particle size distribution in the range of 1 to 20 µm was obtained. This size distribution was achieved due to spray drying and due to controlling the spray drying conditions. It has been found that a higher drying gas flow rate results in the production of smaller particles, while a lower drying gas flow rate results in larger particles. Another parameter that significantly affects the resulting particle size is the content of the solids dissolved in the solution that is spray dried. A higher solids content, i.e., a higher concentration of the solution leads to the formation of larger particles. Spherical particles having the size of 1 to 20 µm are obtained with a drying gas flow rate of 5 to 7 L/min, a dissolved solids content of 15 to 40 mg/mL, using a two-phase nozzle having the diameter of 0.6 to 0.8 mm, an inlet temperature of the drying gas of 90 to 110 °C, an outlet temperature of 60 to 65 °C and a sprayed solution inlet 5 to 7 mL/min. Preferably, the spray drying parameters are: a drying gas flow rate of 6 L/min and a dissolved solids content of 30 mg/mL, a two-phase nozzle with a diameter of 0.7 mm, an inlet temperature of the drying gas of 100 °C, an outlet temperature of 62 °C and a sprayed solution inlet of 6 mL/min. Such narrow size distribution is advantageous in view of subsequent mixing of the particles with other excipients. Change in the flow rate of the drying gas to 8 L/min and/or change in the dissolved solids content to 6 mg/mL while maintaining the values of other parameters resulted in the production of particles of less than 1 µm in size, which were further considered to be somewhat inferior from the technological point of view, because of the increased stickiness of the material and decrease of flow properties.

A polymer approved for pharmaceutical use may be used to form the polymer matrix. Such pharmaceutically acceptable polymers are well known in the art. Factors affecting the choice of polymer include, for example, the chemical purity of the active ingredient, its solubility in the selected polymer, the solubility of the polymer in the selected solvent, the hygroscopicity, etc. Thus, the choice of a suitable polymer or group of polymers may vary in particular cases. Suitable pharmaceutically acceptable polymers include, in particular, homopolymers and copolymers of polyalkylene oxides, such as polyethylene glycol and polypropylene glycol; homopolymers and copolymers of N-vinyl lactams, in particular of N-vinylpyrrolidone, such as polyvinylpyrrolidone (PVP), of vinyl acetates such as Kollidon, and of N-vinylcaprolactam; homopolymers and copolymers of acrylic acids and derivatives thereof; homopolymers and copolymers of methacrylic acid and derivatives thereof, in particular of methyl methacrylate; and cellulose derivatives such as methylcellulose, ethylcellulose, hydroxymethylcellulose, hydroxyethylcellulose (HEC), hydroxypropylcellulose (HPC), hydroxypropylmethylcellulose (HPMC), carboxymethylcellulose, starch derivatives etc. In a preferred embodiment of the invention, the pharmaceutically acceptable polymer is selected from the group consisting of Kollidon VA64, which is chemically a vinylpyrrolidone-vinylacetate copolymer, and PVP K30, which is a polyvinylpyrrolidone.

The weight ratio of empagliflozin to the pharmaceutically acceptable polymer is preferably 1: 1 to 1:10, more preferably 1: 2 to 1: 5, most preferably 1: 2.

The particles are prepared by spray drying to obtain amorphous empagliflozin as a solid solution or a solid dispersion with the pharmaceutically acceptable polymer. Accordingly, the invention also provides a process for preparing the above-described particles, wherein empagliflozin or a pharmaceutically acceptable salt or co-crystal thereof is mixed with the pharmaceutically acceptable polymer and a solvent, and the resulting solution is subjected to spray-drying. The spray-drying conditions are defined in the claims.

The principle of this method is a rapid drying of the sprayed material using a hot gas, preferably nitrogen or air. By dissolving all of the material in a solvent prior to drying and due to the fact that the material is in the form of a solution, a product of uniform density and shape is reliably obtained. Spray drying is carried out on a spray dryer which consists of an atomizer, a gas distributor, a drying chamber, a cyclone separator and a collection container. The starting empagliflozin may be any form of empagliflozin or a pharmaceutically acceptable salt or co-crystal thereof. The crystalline form of empagliflozin and its preparation is well known to those skilled in the art.

The present invention provides particles comprising amorphous empagliflozin prepared by the spray drying method, preferably with a particle size expressed as D(0.9) being less than or equal to 100 µm. In one embodiment, empagliflozin is in the form of a solid solution in a pharmaceutically acceptable polymer, which is preferably polyvinylpyrrolidone, e.g. PVP K30. In another embodiment, the amorphous empagliflozin is in the form of a solid solution with a pharmaceutically acceptable polymer, which is preferably kollidon, eg, Kollidon VA64.

Amorphous empagliflozin prepared by spray drying is suitable for the treatment of type 2 diabetes. It also significantly reduces the risk of heart disease in patients with type 2 diabetes. Accordingly, another object of the invention are the particles of amorphous empagliflozin as described above for use in the treatment or prevention of type 2 diabetes.

The invention further provides a pharmaceutical composition comprising the particles described above. The content of empagliflozin in the pharmaceutical composition is 1 to 100 mg, preferably 5 to 50 mg, even more preferably 10 to 25 mg, most preferably 10 or 25 mg. The content of empagliflozin in the composition is preferably from 1 to 40 % of the total weight of the composition.

The composition may also include one or more excipients that serve in particular as fillers, binders, lubricants, surfactants, disintegrants, colorants, solvents, antimicrobials, or flavor and olfactory additives. Preferably, the composition comprises at least one excipient selected from the group consisting of microcrystalline cellulose, lactose, hydroxypropyl cellulose, sodium croscarmellose, silica, and magnesium stearate.

The pharmaceutical composition can be prepared in virtually any solid dosage form, e.g., in the form of tablets, capsules, powders, pellets, or granules. A preferred dosage form is a capsule, a tablet, or a film-coated tablet. It can be advantageously prepared by mixing the spray-dried material as described above with pharmaceutically acceptable excipients and subjecting it to tabletting, optionally followed by coating.

### Brief Description of Drawings

Fig. 1 shows an XRPD characterization of a) crystalline empagliflozin prepared according to WO2006117359 (lower curve), b) material prepared as described in Example 1 (upper curve).
Fig. 2 shows a scanning electron microscope (SEM) characterization of the material prepared according to Example 1.
Fig. 3 shows a DSC characterization of the material prepared by the process of Example 1.
Fig. 4 shows an XRPD characterization of a) crystalline empagliflozin according to WO2006117359 (lower curve), b) material prepared as described in Example 2 (upper curve).
Fig. 5 shows a scanning electron microscope (SEM) characterization of the solid solution prepared according to Example 2.
Fig. 6 shows a DSC characterization of the material prepared by the process of Example 2.

### Examples of carrying out the Invention

The following examples are intended to illustrate and further explain the invention, and should not be construed as limiting the scope of the protection which is defined by the claims.

### Example 1

10 g of empagliflozin in crystalline form were dissolved in 1000 mL of pure ethanol. 20 g of Kollidon VA64 was added and dissolved in the resulting solution. The resulting solution thus had the solids content of 30 mg/mL and was spray dried in a Buchi Mini Spray Dryer B-290 sprayer equipped with a 0.7 mm diameter two-phase nozzle. The drying gas was air, and the gas flow rate was 6 L/min, the gas inlet temperature was 100 °C and the outlet temperature was 62 °C. Injection speed of the solution into the spray dryer was 6 mL/min. Yield of the spray-dried material was 80 %, relative to the solids contained in the starting solution. Such yield is considered excellent for a small-scale spray-dryer experiment.

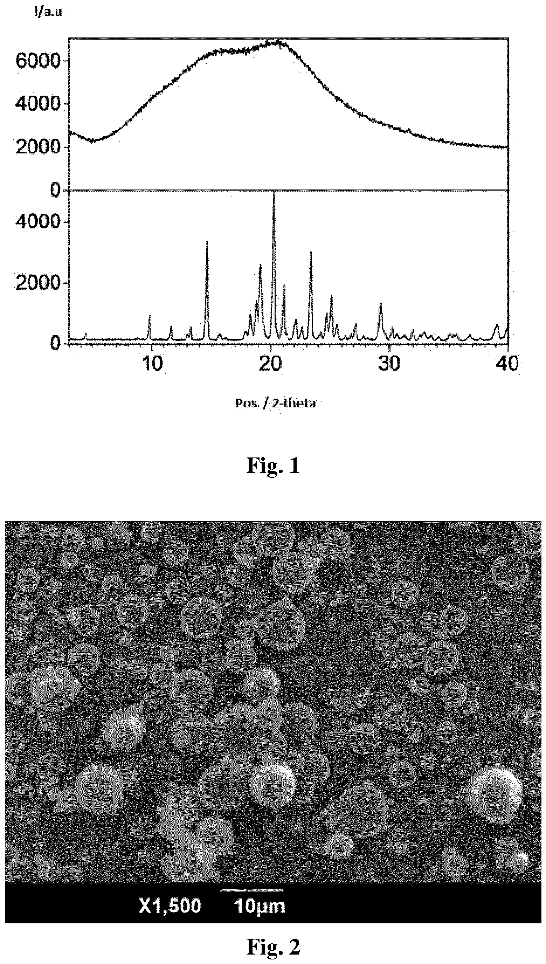

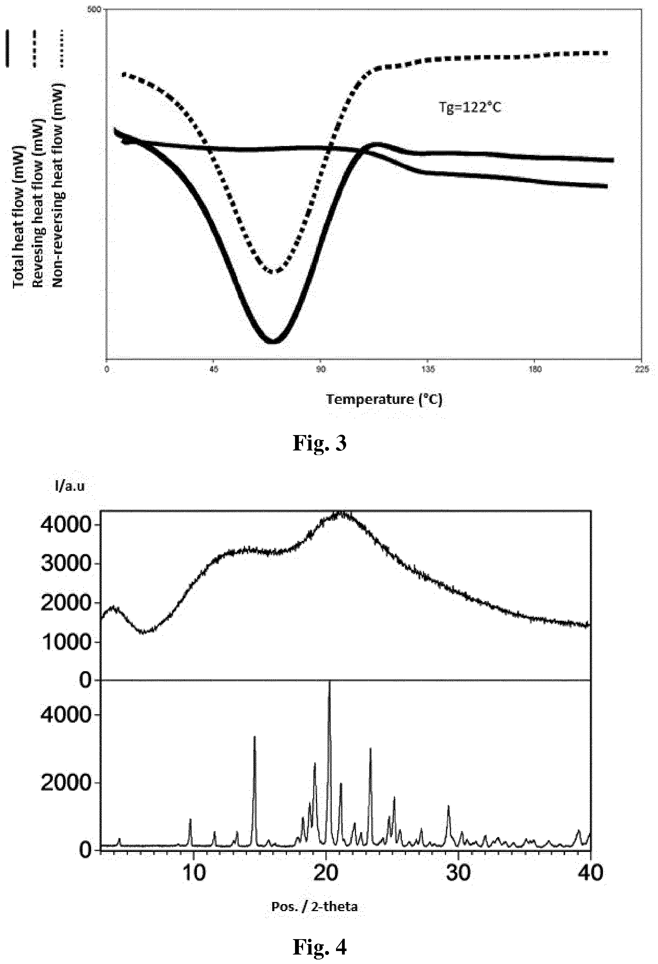

The prepared material was characterized by X-ray powder diffraction (XRPD), see Fig. 1. The results show that it is an amorphous material. Fig. 2 represents a scanning electron microscopy characterization, which shows that the resulting material is in the form of spherical particles with an average size of about 5 µm and size distribution from 1 to 10 µm. The particle size was determined by image analysis of electron microscope images. Differential scanning calorimetry (DSC) with a heating rate of 5 °C/min (amplitude = 0.8 °C, period = 60 s) was used to determine whether the product is a solid solution of amorphous empagliflozin with polymer or an amorphous dispersion of empagliflozin in polymer. The results in Fig. 3 show that the product had a glass transition temperature (Tg = 122 °C). Thus, empagliflozin is in the form of a solid solution.

### Example 2

10 g of empagliflozin in crystalline form were dissolved in 1000 mL of pure ethanol. 20 g of PVP K30 was added and dissolved in the resulting solution. The resulting solution thus had the solids content of 30 mg/mL and was spray dried in a Buchi Mini Spray Dryer B-290 sprayer equipped with a 0.7 mm diameter two-phase nozzle. The drying gas was air, and the gas flow rate was 6 L/min, the gas inlet temperature was 100 °C and the outlet temperature was 62 °C. Injection speed of the solution into the spray dryer was 6 mL/min. Yield of the spray-dried material was 80 %, relative to the solids contained in the starting solution. Such yield is considered excellent for a small-scale spray-dryer experiment.

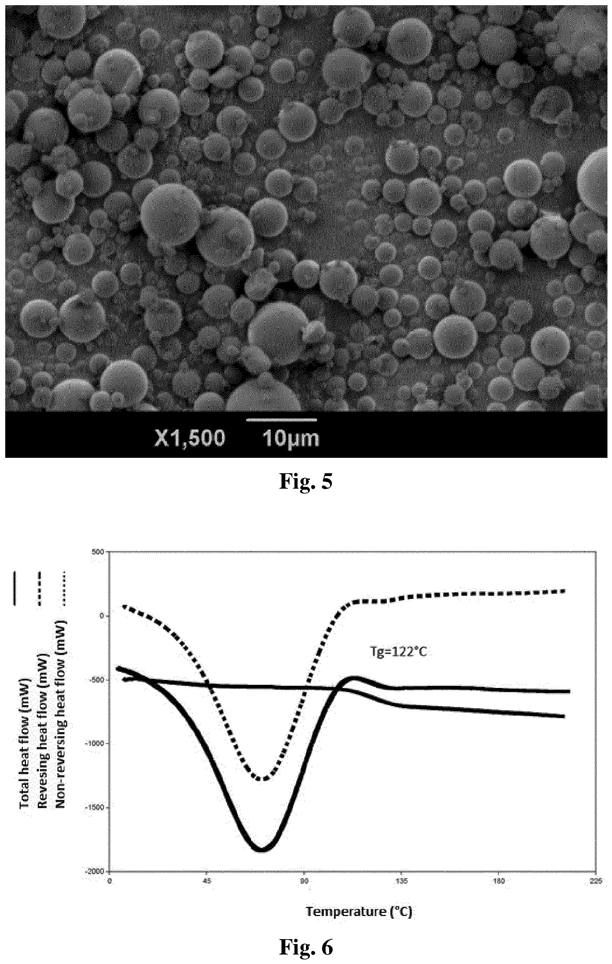

The prepared material was characterized by X-ray powder diffraction (XRPD), see Fig. 4. The results show that it is an amorphous material. Fig. 5 represents a scanning electron microscopy characterization, which shows that the resulting material is in the form of spherical particles with an average size of about 10 µm and size distribution from 5 to 20 µm. The particle size was determined by image analysis of electron microscope images. Differential scanning calorimetry (DSC) with a heating rate of 5 °C/min (amplitude = 0.8 °C, period = 60 s) was used to determine whether the product is a solid solution of amorphous empagliflozin with polymer or an amorphous dispersion of empagliflozin in polymer. The results in Fig. 6 show that the product had one glass transition temperature (Tg = 122 °C). Thus, empagliflozin is in the form of a solid solution.

### Example 3

10 g of empagliflozin in crystalline form were dissolved in 1000 mL of pure ethanol. 20 g of Kollidon VA64 was added and dissolved in the resulting solution. The resulting solution thus had the solids content of 30 mg/mL and was spray dried in a Buchi Mini Spray Dryer B-290 sprayer equipped with a 0.7 mm diameter two-phase nozzle. The drying gas was nitrogen, and the gas flow rate was 6 L/min, the gas inlet temperature was 100 °C and the outlet temperature was 62 °C. Injection speed of the solution into the spray dryer was 6 mL/min. Yield of the spray-dried material was 80 %, relative to the solids contained in the starting solution. Such yield is considered excellent for a small-scale spray-dryer experiment.

The thus prepared material was characterized by X-ray powder diffration (XRPD) method. The results show that the product is amorphous.

### Example 4

10 g of empagliflozin in crystalline form were dissolved in 1000 mL of pure ethanol. 20 g of PVP K30 was added and dissolved in the resulting solution. The resulting solution thus had the solids content of 30 mg/mL and was spray dried in a Buchi Mini Spray Dryer B-290 sprayer equipped with a 0.7 mm diameter two-phase nozzle. The drying gas was nitrogen, and the gas flow rate was 6 L/min, the gas inlet temperature was 100 °C and the outlet temperature was 62 °C. Injection speed of the solution into the spray dryer was 6 mL/min. Yield of the spray-dried material was 80 %, relative to the solids contained in the starting solution. Such yield is considered excellent for a small-scale spray-dryer experiment.

The prepared material was characterized by X-ray powder diffration (XRPD) method. The results show that the product is amorphous.

### Example 5

The spray-dried particles prepared in Example 2 were mixed with microcrystalline cellulose, lactose, hydroxypropyl cellulose (HPC), croscarmellose sodium, and magnesium stearate. The mixture was homogenized and tableted into tablets containing 10.0 mg or 25.0 mg empagliflozin. The tablets showed excellent properties - tabletability, strength, etc. The composition of the individual tablets is shown in Tables 1 and 2.

**Table 1 - strength 10.0 mg**

| Component | Function | Amount |
|---|---|---|
| Spray-dried particles | Polymer matrix with active ingredient | 30 mg |
| Microcrystalline cellulose | Filler | 67.5 mg |
| Lactose | Filler | 149.6 mg |
| HPC | Binder | 5.2 mg |
| Sodium crosscarmellose | Disintegrant | 5.2 mg |
| Magnesium stearate | Lubricant | 2.6 mg |

**Table 2 - strength 25.0 mg**

| Component | Function | Amount |
|---|---|---|
| Spray-dried particles | Polymer matrix with active ingredient | 75 mg |
| Microcrystalline cellulose | Filler | 47.5 mg |
| Lactose | Filler | 69.5 mg |
| HPC | Binder | 5.2 mg |
| Sodium crosscarmellose | Disintegrant | 5.2 mg |
| Magnesium stearate | Lubricant | 2.6 mg |

## Claims

1. Particles containing amorphous empagliflozin and a pharmaceutically acceptable polymer, said particles having the size expressed as D(0.9) less than or equal to 100 µm, determined by image analysis of scanning electron microscope images, and said particles being obtainable by spray drying at drying gas flow rate from 5 to 7 L/min, at a content of dissolved solids in a solution from 15 to 40 mg/mL, using a two-phase nozzle having a diameter from 0.6 to 0.8 mm, at an inlet drying gas temperature from 90 to 110 °C, at an outlet temperature from 60 to 65 °C, and at a solution injection rate from 5 to 7 mL/min.

2. The particles according to claim 1, having the size expressed as D(0.9) less than or equal to 50 µm, more preferably less than or equal to 20 µm.

3. The particles according to claim 1 or claim 2, having the size distribution in the range of 1 to 20 µm, wherein the particles are spherical.

4. The particles according to any one of the preceding claims, wherein amorphous empagliflozin is in the form of a solid solution with the pharmaceutically acceptable polymer.

5. The particles according to any one of the preceding claims, wherein the pharmaceutically acceptable polymer is selected from the group consisting of homopolymers and copolymers of polyalkylene oxides, polyvinylpyrrolidones, vinylpyrrolidones, vinylacetate, N-vinyllactams, acrylic acid, methacrylic acid, methylmethacrylate and cellulose derivatives.

6. The particles according to any one of the preceding claims, wherein the pharmaceutically acceptable polymer is selected from the group consisting of homopolymers and copolymers of polyvinylpyrrolidone.

7. The particles according to any one of the preceding claims, wherein the weight ratio of empagliflozin and the pharmaceutically acceptable polymer is from 1:1 to 1:10, preferably 1:2.

8. A method of preparation of the particles according to any one of the preceding claims, **characterized in that** empagliflozin or a pharmaceutically acceptable salt thereof or a co-crystal thereof is mixed with a pharmaceutically acceptable polymer and a solvent, and the resulting solution is subjected to spray-drying at drying gas flow rate from 5 to 7 L/min, at a content of dissolved solids in the solution from 15 to 40 mg/mL, using a two-phase nozzle having a diameter from 0.6 to 0.8 mm, at an inlet drying gas temperature from 90 to 110 °C, at an outlet temperature from 60 to 65 °C, and at a solution injection rate from 5 to 7 mL/min.

9. The method according to claim 8, wherein spray drying is carried out at drying gas flow rate of 6 L/min, at a content of dissolved solids in the solution 30 mg/mL, using a two-phase nozzle having the diameter of 0.7 mm, at an inlet drying gas temperature of 100 °C, at an outlet temperature 62 °C, and at a solution injection rate of 6 mL/min.

10. The particles according to any one of claims 1 to 7 for use in the treatment or prevention of type 2 diabetes.

11. A pharmaceutical composition, **characterized in that** it contains particles according to any one of claims 1 to 7, and at least one pharmaceutically acceptable excipient.

12. The pharmaceutical composition according to claim 11, wherein the content of empagliflozin is from 1 to 100 mg, preferably from 10 to 25 mg, more preferably 10 or 25 mg, in unit dose.

13. The pharmaceutical composition according to claim 11 or 12, which is in the form of a capsule, a tablet, or a film-coated tablet.

14. The pharmaceutical composition according to any one of claims 11 to 13, wherein the excipient is selected from the group consisting of lactose, microcrystalline cellulose, hydroxypropyl cellulose, sodium crosscarmelose, and magnesium stearate.

## Patentansprüche

1. Partikel, die amorphes Empagliflozin und ein pharmazeutisch verträgliches Polymer enthalten, wobei die Partikel eine Größe haben, die ausgedrückt wird als D(0,9) kleiner oder gleich 100 µm, bestimmt durch Bildanalyse von Rasterelektronenmikroskopbildern, und die Partikel erhältlich sind durch Sprühtrocknung bei Trocknungsgasströmungsgeschwindigkeit von 5 bis 7 l/min bei einem Gehalt an gelösten Feststoffen in einer Lösung von 15 bis 40 mg/ml unter Verwendung einer Zweiphasendüse mit einem Durchmesser von 0,6 bis 0,8 mm bei einem Einlasstrocknungsgastemperatur von 90 bis 110 °C, bei einer Auslasstemperatur von 60 bis 65 °C und bei einer Lösungsinjektionsgeschwindigkeit von 5 bis 7 ml/min.

2. Partikel nach Anspruch 1, wobei die Partikel eine Größe haben, die ausgedrückt wird als D(0,9) kleiner oder gleich 50 µm, bevorzugter kleiner oder gleich 20 µm.

3. Partikel nach Anspruch 1 oder Anspruch 2 mit einer Größenverteilung im Bereich von 1 bis 20 µm, wobei die Partikel kugelförmig sind.

4. Partikel nach einem der vorhergehenden Ansprüche, wobei amorphes Empagliflozin in Form einer festen Lösung mit dem pharmazeutisch verträglichen Polymer vorliegt.

5. Partikel nach einem der vorhergehenden Ansprüche, wobei das pharmazeutisch verträgliche Polymer ausgewählt ist aus der Gruppe bestehend aus Homopolymeren und Copolymeren von Polyalkylenoxiden, Polyvinylpyrrolidonen, Vinylpyrrolidonen, Vinylacetat, N-Vinyllactamen, Acrylsäure, Methacrylsäure, Methylmethacrylat und Cellulose Derivate.

6. Partikel nach einem der vorhergehenden Ansprüche, wobei das pharmazeutisch verträgliche Polymer ausgewählt ist aus der Gruppe bestehend aus Homopolymeren und Copolymeren von Polyvinylpyrrolidon.

7. Partikel nach einem der vorhergehenden Ansprüche, wobei das Gewichtsverhältnis von Empagliflozin und dem pharmazeutisch verträglichen Polymer 1:1 bis 1:10, vorzugsweise 1:2, beträgt.

8. Ein Verfahren zur Herstellung der Partikel nach einem der vorhergehenden Ansprüche, das **dadurch gekennzeichnet ist, dass** Empagliflozin oder ein pharmazeutisch verträgliches Salz davon oder ein Co-Kristall davon mit einem pharmazeutisch verträglichen Polymer mit einem Lösungsmittel gemischt wird und die resultierende Lösung wird einer Sprühtrocknung unterzogen bei einer Trocknungsgasströmungsgeschwindigkeit von 5 bis 7 l/min bei einem Gehalt an gelösten Feststoffen in der Lösung von 15 bis 40 mg/ml unter Verwendung einer Zweiphasendüse mit einem Durchmesser von 0,6 bis 0,8 mm bei einer Einlasstrocknungsgastemperatur von 90 bis 110 °C, bei einer Auslasstemperatur von 60 bis 65 °C und bei einer Lösungsinjektionsgeschwindigkeit von 5 bis 7 ml/min.

9. Verfahren nach Anspruch 8, wobei das Sprühtrocknen durchgeführt wird bei einer Trocknungsgasströmungsgeschwindigkeit von 61/min bei einem Gehalt an gelösten Feststoffen in der Lösung von 30 mg/ml unter Verwendung einer Zweiphasendüse mit dem Durchmesser von 0,7 mm bei einer Einlasstrocknungsgastemperatur von 100 °C, einer Auslasstemperatur von 62 °C und einer Lösungsinjektionsgeschwindigkeit von 6 ml/min.

10. Partikel nach einem der Ansprüche 1 bis 7 zur Verwendung bei der Behandlung oder Vorbeugung von Typ-2-Diabetes.

11. Pharmazeutische Zusammensetzung, die **dadurch gekennzeichnet ist, dass** sie Partikel nach einem der Ansprüche 1 bis 7 und mindestens einen pharmazeutisch verträglichen Hilfsstoff enthält.

12. Pharmazeutische Zusammensetzung nach Anspruch 11, wobei der Gehalt an Empagliflozin 1 bis 100 mg, vorzugsweise 10 bis 25 mg, bevorzugter 10 oder 25 mg, in Einheitsdosis beträgt.

13. Pharmazeutische Zusammensetzung nach Anspruch 11 oder 12, die in Form einer Kapsel, einer Tablette oder einer Filmtablette vorliegt.

14. Pharmazeutische Zusammensetzung nach einem der Ansprüche 11 bis 13, wobei der Hilfsstoff ausgewählt ist aus der Gruppe bestehend aus Lactose, mikrokristalliner Cellulose, Hydroxypropylcellulose, Natriumkreuzcarmelose und Magnesiumstearat.

## Revendications

1. Particules contenant de l'empagliflozine amorphe et un polymère pharmaceutiquement acceptable, lesdites particules ayant la taille exprimée en D(0,9) inférieure ou égale à 100 µm, déterminée par analyse d'images au microscope électronique à balayage, et lesdites particules pouvant être obtenues par séchage par atomisation à une vitesse du gaz de séchage de 5 à 7 L/min, à une teneur en solides dissous dans une solution de 15 à 40 mg/mL, utilisant une buse biphasée d'un diamètre de 0,6 à 0,8 mm, à une température d'entrée du gaz de séchage de 90 à 110 °C, à une température de sortie de 60 à 65 °C et à une vitesse d'injection de solution de 5 à 7 mL/min.

2. Particules selon la revendication 1, ayant la taille exprimée en D(0,9) inférieure ou égale à 50 µm, de préférence inférieure ou égale à 20 µm.

3. Particules selon la revendication 1 ou la revendication 2, ayant la distribution de taille dans la gamme de 1 à 20 µm, où les particules sont sphériques.

4. Particules selon l'une quelconque des revendications précédentes, où l'empagliflozine amorphe se présente sous la forme d'une solution solide avec le polymère pharmaceutiquement acceptable.

5. Particules selon l'une quelconque des revendications précédentes, où le polymère pharmaceutiquement acceptable est choisi dans le groupe constitué par les homopolymères et copolymères d'oxydes de polyalkylène, les polyvinylpyrrolidones, les vinylpyrrolidones, l'acétate de vinyle, les N-vinyllactames, l'acide acrylique, l'acide méthacrylique, le méthylméthacrylate et les dérivés de cellulose.

6. Particules selon l'une quelconque des revendications précédentes, où le polymère pharmaceutiquement acceptable est choisi dans le groupe constitué par les homopolymères et copolymères de polyvinylpyrrolidone.

7. Particules selon l'une quelconque des revendications précédentes, où le rapport pondéral de l'empagliflozine et du polymère pharmaceutiquement acceptable est de 1:1 à 1:10, de préférence 1:2.

8. Procédé de préparation des particules selon l'une quelconque des revendications précédentes, **caractérisé en ce que** l'empagliflozine ou un sel pharmaceutiquement acceptable de celle-ci ou un co-cristal de celle-ci est mélangée avec un polymère pharmaceutiquement acceptable et un solvant, et le solution résultant est soumis à un séchage par atomisation à une vitesse du gaz de séchage de 5 à 7 L/min, à une teneur en solides dissous dans une solution de 15 à 40 mg/mL, utilisant une buse biphasée d'un diamètre de 0,6 à 0,8 mm, à une température d'entrée du gaz de séchage de 90 à 110 °C, à une température de sortie de 60 à 65 °C et à une vitesse d'injection de solution de 5 à 7 mL/min.

9. Procédé selon la revendication 8, où le séchage par atomisation est effectué à une vitesse de gaz de séchage de 6 L/min, à une teneur en solides dissous dans la solution de 30 mg/mL, utilisant une buse biphasée d'un diamètre de 0,7 mm, à une température d'entrée du gaz de séchage de 100 °C, à une température de sortie de 62 °C et à une vitesse d'injection de solution de 6 mL/min.

10. Particules selon l'une quelconque des revendications 1 à 7 pour utilisation dans le traitement ou la prévention du diabète de type 2.

11. Composition pharmaceutique, **caractérisée en ce qu'**elle contient des particules selon l'une quelconque des revendications 1 à 7, et au moins un excipient pharmaceutiquement acceptable.

12. Composition pharmaceutique selon la revendication 11, dans laquelle la teneur en empagliflozine est de 1 à 100 mg, de préférence de 10 à 25 mg, plus préférablement de 10 ou 25 mg, en dose unitaire.

13. Composition pharmaceutique selon la revendication 11 ou 12, qui se présente sous la forme d'une capsule, d'un comprimé ou d'un comprimé pelliculé.

14. Composition pharmaceutique selon l'une quelconque des revendications 11 à 13, dans laquelle l'excipient est choisi dans le groupe constitué par le lactose, la cellulose microcristalline, l'hydroxypropylcellulose, le crosscarmelose de sodium et le stéarate de magnésium.
